(19) **Europäisches Patentamt / European Patent Office / Office européen des brevets**

(11) **EP 4 406 478 A1**

(12) **EUROPEAN PATENT APPLICATION**
published in accordance with Art. 153(4) EPC

(43) Date of publication:
**31.07.2024 Bulletin 2024/31**

(21) Application number: **22873190.7**

(22) Date of filing: **22.09.2022**

(51) International Patent Classification (IPC):
*A61B 5/276* (2021.01)    *A61B 5/327* (2021.01)
*A61B 5/319* (2021.01)    *A61B 5/346* (2021.01)
*A61B 5/28* (2021.01)    *A61B 5/00* (2006.01)

(52) Cooperative Patent Classification (CPC):
**A61B 5/00; A61B 5/276; A61B 5/28; A61B 5/319; A61B 5/327; A61B 5/346**

(86) International application number:
**PCT/KR2022/014204**

(87) International publication number:
**WO 2023/048486 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2021 KR 20210126783**
**20.09.2022 KR 20220118468**

(71) Applicant: **Medical AI Co., Ltd.**
**Seoul 06302 (KR)**

(72) Inventors:
• **KWON, Joonmyoung**
**Seoul 06302 (KR)**
• **LEE, Byeongtak**
**Seoul 06302 (KR)**

(74) Representative: **Patentanwaltskanzlei Matschnig & Forsthuber OG**
**Biberstraße 22**
**Postfach 36**
**1010 Wien (AT)**

(54) **METHOD, PROGRAM AND DEVICE FOR CORRECTING ERROR OF ELECTROCARDIOGRAM SIGNAL**

(57) A method of correcting an error in an electrocardiogram signal, which is performed by a computing device, according to an embodiment of the present disclosure includes: acquiring electrocardiogram signals; estimating an error in the acquired electrocardiogram signals based on the correlations between leads for the measurement of the acquired electrocardiogram signals; and correcting an electrocardiogram signal, estimated to have the error, based on correction information for the correction of an error in an electrocardiogram signal.

FIG. 2

EP 4 406 478 A1

**Description**

**Technical Field**

The present disclosure relates to a method of correcting an error in an electrocardiogram signal, and more particularly to a method that detects an error occurring in the process of measuring electrocardiogram signals based on the correlations between the electrocardiogram signals and corrects an electrocardiogram signal.

**Background Art**

Clinical examination, diagnostic imaging, and the like are used to check for abnormalities in the heart. In particular, for the early diagnosis of heart disease, there is widely used a method of measuring electrocardiograms, displaying measured electrocardiogram signals in the form of a graph, and determining the presence/absence of abnormalities in a patient's heart based on the graph.

Electrocardiography (ECG) records the active currents generated in the myocardium as the heart beats in the form of waves. A 12-lead electrocardiogram method measures signals with 10 electrodes attached to a patient's body. In this case, among the 10 electrodes, 6 precordial lead electrodes (V1 to V6) are attached to specific anatomical locations according to a unipolar precordial lead method. Furthermore, the residual 4 electrodes are attached to a patient's limbs, that is, the left hand and foot and the right hand and foot, respectively. However, the electrodes attached to the left hand and foot and the right hand and foot, respectively, may be attached to the upper left and right sides and lower left and right sides of the chest in some cases.

In general, in order to measure electrocardiogram signals, it is necessary to find the locations to which 10 electrocardiogram electrodes will be attached on a patient's body and attach them one by one. However, it is not easy to find the exact locations to which 10 electrodes will be attached on a patient. Furthermore, even when used by skilled personnel such as medical staff, the electrodes are often attached incorrectly, so that signals that cannot be diagnosed may be measured or incorrect diagnoses may occur.

Therefore, in order to measure correct signals with electrocardiogram electrodes attached, there is a need for a method that, even when the locations of electrocardiogram electrodes are reversed, can determine this situation, correct the signal in which lead reversal has occurred, and rapidly provide normal electrocardiogram signals.

**Disclosure**

**Technical Problem**

The present disclosure has been conceived in response to the above-described background technology, and an object of the present disclosure is to provide a method that determines an error, such as a lead reversal case resulting from the misplacement of electrocardiogram electrodes, based on electrocardiogram signals and corrects a signal determined to have the error to a correct a signal.

An object of the present disclosure is to provide a method that classifies the electrocardiogram signal, in which an error such as a lead reversal case has occurred, by using a trained deep learning model.

However, the objects to be accomplished by the present disclosure are not limited to the objects mentioned above, and other objects not mentioned may be clearly understood based on the following description.

**Technical Solution**

According to an embodiment of the present disclosure for accomplishing the above-described objects, there is disclosed a method of correcting an error in an electrocardiogram signal, the method being performed by a computing device including at least one processor, the method including: acquiring electrocardiogram signals; estimating an error in the acquired electrocardiogram signals based on the correlations between leads for the measurement of the acquired electrocardiogram signals; and correcting an electrocardiogram signal, estimated to have the error, based on correction information for the correction of an error in an electrocardiogram signal.

Alternatively, estimating the error in the acquired electrocardiogram signals may include: generating a correlation matrix representing the electrocardiogram signal relationships between the leads based on the acquired electrocardiogram signals; and detecting an electrocardiogram signal, in which lead reversal has occurred, among the acquired electrocardiogram signals based on the correlation matrix.

Alternatively, detecting the electrocardiogram signal in which lead reversal has occurred may include classifying the electrocardiogram signal, in which the lead reversal has occurred, among the acquired electrocardiogram signals by inputting the correlation matrix to a pre-trained first neural network model.

Alternatively, estimating the error in the acquired electrocardiogram signals may include classifying the electrocardiogram signal, in which the lead reversal has occurred, among the electrocardiogram signals by inputting the electrocardiogram signals to a pre-trained second neural network model.

Alternatively, estimating the error in the acquired electrocardiogram signals may include extracting target signals for the analysis of the correlations from the acquired electrocardiogram signals by using a band-pass filter.

Alternatively, the correction information may include a correction table representing the conversion relationships between the electrocardiogram signals of normal leads and the electrocardiogram signals in which lead reversal has occurred.

Alternatively, electrodes attached to a human body to acquire the electrocardiogram signals may include one or more of a plurality of electrodes attached to the distal ends of the limbs and a plurality of electrodes attached to preset locations on the chest.

Alternatively, the acquired electrocardiogram signals may include one or more of a first electrocardiogram signal generated based on a first electrode corresponding to the right arm and a second electrode corresponding to the left arm, a second electrocardiogram signal generated based on the first electrode and a third electrode corresponding to the left leg, a third electrocardiogram signal generated based on the second electrode and the third electrode, and precordial electrocardiogram signals corresponding to a plurality of precordial electrodes, respectively.

According to an embodiment of the present disclosure for accomplishing the above-described object, there is disclosed a computer program stored in a computer-readable storage medium, the computer program performing the operations of correcting an error in an electrocardiogram signal when executed on one or more processors, wherein the operations include the operations of: acquiring electrocardiogram signals; estimating an error in the acquired electrocardiogram signals based on the correlations between leads for the measurement of the acquired electrocardiogram signals; and correcting an electrocardiogram signal, estimated to have the error, based on correction information for the correction of an error in an electrocardiogram signal.

According to an embodiment of the present disclosure for accomplishing the above-described object, there is disclosed a computing device for correcting an error in an electrocardiogram signal, the computing device including: a processor including at least one core; memory including program codes that are executable on the processor; and a network unit configured to acquire electrocardiogram signals; wherein the processor acquires electrocardiogram signals, estimates an error in the acquired electrocardiogram signals based on the correlations between leads for the measurement of the acquired electrocardiogram signals, and corrects an electrocardiogram signal, estimated to have the error, based on correction information for the correction of an error in an electrocardiogram signal.

**Advantageous Effects**

The present disclosure may detect a lead reversal case, restore a correct electrocardiogram signal and provide it to a user even when electrodes for the measurement of electrocardiogram signals are misplaced.

Through the present disclosure, when an electrocardiogram signal is measured incorrectly, misdiagnosis may be prevented by specifically identifying and correcting a lead reversal case.

In the present disclosure, when lead reversal occurs due to the misplacement of an electrocardiogram electrode, an electrocardiogram signal can be corrected and provided to a user without additional measurement.

**Description of Drawings**

FIG. 1 is a block diagram of a computing device according to an embodiment of the present disclosure;
FIG. 2 is a block diagram showing a process of correcting an error in an electrocardiogram signal according to an embodiment of the present disclosure;
FIG. 3 is a diagram illustrating electrocardiogram electrode attachment locations and leads corresponding to these locations according to an embodiment of the present disclosure;
FIG. 4 is a table illustrating signals that are generated during lead reversal according to an embodiment of the present disclosure;
FIG. 5 is a diagram illustrating a neural network model according to an embodiment of the present disclosure; and
FIG. 6 is a flowchart showing a method of correcting an error in an electrocardiogram signal according to an embodiment of the present disclosure.

**Mode for Invention**

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquisition" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

"Data" used herein may include "images," signals, etc. The term "image" used herein may refer to multidimensional data composed of discrete image elements. In other words, "image" may be understood as a term referring to a digital representation of an object that can be seen by the human eye. For example, "image" may refer to multidimensional data composed of elements corresponding to pixels in a two-dimensional image. "Image" may refer to multidimensional data composed of elements corresponding to voxels in a three-dimensional image.

"Lead reversal" used herein may refer to the misplacement of a lead, and may occur when an electrode for the measurement of an electrocardiogram is attached to an incorrect location. For example, in the case of the lead reversal in which one of the limb electrodes other than the neutral electrode is misplaced, a specific lead signal may be changed into another lead signal, another specific lead signal may be inverted or rotated, or still another specific lead signal may not be changed. For example, when the neutral electrode of the limb electrodes is misplaced, not only a limb lead signal but also a precordial lead signal may be distorted. In this case, the lead signal may appear as any other lead signal, or may appear reduced in size to almost zero.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

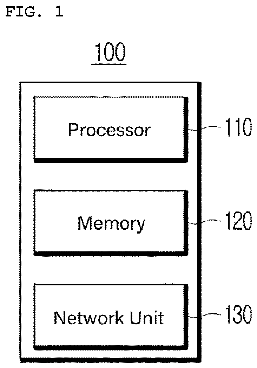

FIG. 1 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 1, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may detect an error in an electrocardiogram signal that occurs when an electrode for the measurement of an electrocardiogram is placed at an incorrect location. An electrode being placed at an incorrect location may be understood as an electrode for measuring an electrocardiogram signal for a specific lead being placed outside of its correct location. When an electrode is placed at an incorrect location, the probability that an undiagnosable electrocardiogram signal will be measured or that a misdiagnosis will occur due to a measured electrocardiogram signal increases. To prevent this problem, the processor 110 may detect an error in an electrocardiogram signal that occurs due to the displacement of an electrode at an incorrect location based on measured electrocardiogram signals.

For example, the processor 110 may detect a lead reversal case resulting from the misplacement of an electrode by analyzing electrocardiogram signals. More specifically, the processor 110 may analyze the correlations between leads for the measurement of electrocardiogram signals based on electrocardiogram signals. In this case, the correlations between leads may be understood as analysis results indicating the correlations between the electrocardiogram signals of leads based on measured signals for the respective leads. The processor 110 may detect a lead reversal case using the correlations between leads analyzed based on the measured electrocardiogram signals. Based on the correlations between leads, the processor 110 may detect the lead whose signal was measured in a reversed form due to the misplacement of an electrode at an incorrect location. In this case, a calculation process for the detection of a lead reversal case may be performed according to a rule-based logic defined by program code, or may be performed based on a deep learning algorithm based on program code. Through this calculation process, the processor 110 may easily detect a reversal case that has occurred in the measurement signal of each of all the leads used to measure electrocardiogram signals.

The processor 110 may not only detect an error in an electrocardiogram signal, but also correct the detected error in the electrocardiogram signal. The processor 110 may remedy the detected error in the electrocardiogram signal based on predetermined correction information to correct the error in the electrocardiogram signal. In this case, the correction information may be the information that is predefined to convert a signal for each lead measured when an electrode is placed at an incorrect location into a signal for each lead measured when the electrode is placed at a normal location. That is, the processor 110 may convert the electrocardiogram signal in which an error has been detected into a signal which is measured when an electrode is placed at a normal location by using the correction information.

The error detection and correction process for electrocardiogram signals performed through the processor 110 may minimize the resources of a hospital environment required to acquire electrocardiogram signals that can be normally used for the diagnosis of disease and the like. For example, even when signals are measured with electrodes incorrectly set for the measurement of electrocardiogram signals, the above-described error detection and correction process may minimize the inconvenience of the work for resetting electrodes and measuring signals again.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data necessary for the processor 110 to perform computation, the combination of data, and program codes executable on the processor 110. For example, the memory 120 may store electrocardiogram signals or electrocardiogram data received through the network unit 130, which will be described later. The memory 120 may store program codes configured to operate the neural network model to receive electrocardiogram signals or various types of data related to electrocardiogram signals and perform learning, program codes configured to operate the neural network model to receive electrocardiogram signals or various types of data related to electrocardiogram signals and perform inference in accordance with the purpose of use of the computing device 100, processed data generated as program codes are executed, etc.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data necessary for the processor 110 to perform computation through wired/wireless communication with any system or client or the like. Furthermore, the network unit 130 may transmit data generated through the computation of the processor 110 through wired/wireless communication with any system or client or the like. For example, the network unit 130 may receive electrocardiogram-related data through communication with an electrocardiogram signal measurement device, a database within a hospital environment, a cloud server, or a computing device. The network unit 130 may transmit the output data of the neural network model, intermediate data and processed data acquired from the computation process of the processor 110, etc. through communication with the above-described database, server, or computing device.

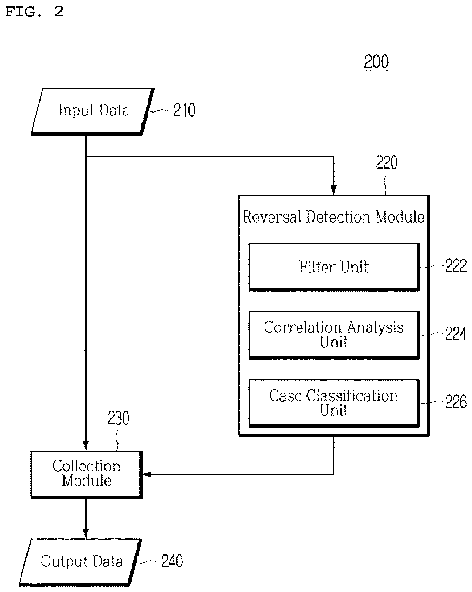

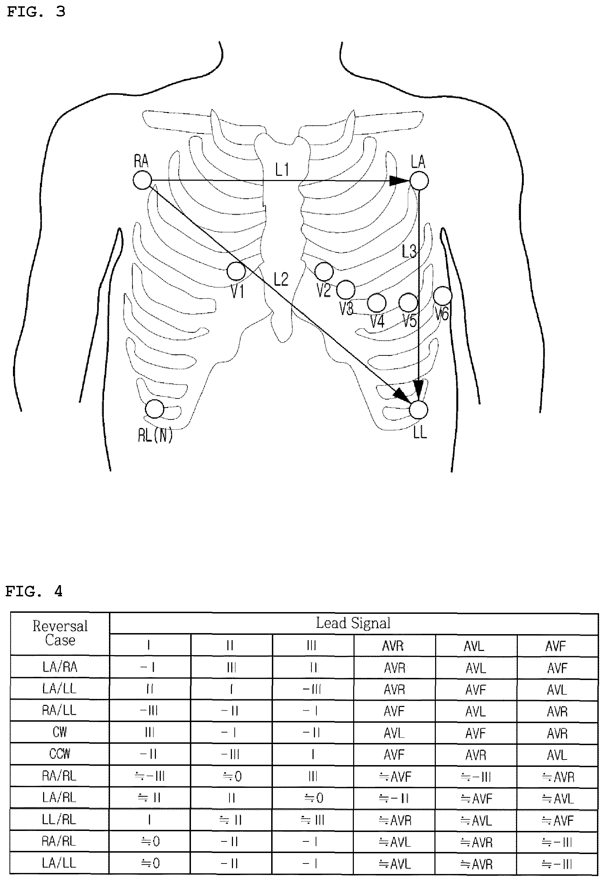

FIG. 2 is a block diagram showing a process of correcting an error in an electrocardiogram signal according to an embodiment of the present disclosure. Furthermore, FIG. 4 is a table illustrating signals that are generated during lead reversal according to an embodiment of the present disclosure.

Referring to FIG. 2, the computing device 200 for correcting an error in an electrocardiogram signal includes a reversal detection module 220 configured to detect an error in input data 210 and a correction module 230 configured to correct the error in the input data 210. In the present disclosure, the input data 210 may include an electrocardiogram signal corresponding to at least one lead. The input data 210 may be generated in an electrocardiogram measurement device and transmitted to the computing device 200. When the computing device 200 includes electrodes for the measurement of electrocardiogram signals, the input data 210 may be generated directly in the computing device 200.

The reversal detection module 220 may include a filter unit 222, a correlation analysis unit 224, and a case classification unit 226.

The filter unit 222 may filter out noise in the input data 210 to facilitate the analysis of the input data 210. For example, the filter unit 222 may include a band-pass filter configured to pass only components in a specific frequency band to be analyzed in the input data 210 therethrough. The band-pass filter may only pass signals in the band of 0.5 Hz to 50 Hz in the input data 210 therethrough. However, the band of 0.5 Hz to 50 Hz is only one example, and thus, the present disclosure is not limited to these values. The filter unit 222 may further include a notch filter configured to reduce mains hum, which is power noise generated from a power cable and/or the like.

The correlation analysis unit 224 may analyze the correlation for each lead. The correlation analysis of the correlation analysis unit 224 may be performed based on the input data 210 or the output data of the filter unit 222. The correlation analysis unit 224 may generate a correlation matrix of size $N \times N$ indicating the correlations between the electrocardiogram signals of leads by analyzing the relationships between the electrocardiogram signals of the leads for the individual leads. For example, when the input data 210 includes 12-lead electrocardiogram signals, the correlation analysis unit 224 may analyze the morphological correlations between 12 leads and generate a correlation matrix of size $12 \times 12$. For a calculation process for the generation of a correlation matrix of size $12 \times 12$, reference may be made to Equation 1 below:

$$X = X - E[X] \in R^{T \cdot C} \quad (X \in R^{T \cdot C}, \ E[X] \in R^{C})$$

$$A = X^{T}X \in R^{C \cdot C} \tag{1}$$

In Equation 1, X may denote an electrocardiogram signal, C may denote a channel or lead, T may denote time, and A may denote an adjacency matrix. E [X] may be the expected value of the random variable X.

As described above, the correlation analysis unit 224 may organize the correlations between all the leads into one matrix based on electrocardiogram signals. The correlation analysis unit 224 may represent the signal relationships between all the leads as a correlation matrix so that the case classification unit 226 can easily interpret complex signal relationships. That is, the case classification unit 226 may easily perform the characteristic interpretation of signals for the respective leads using the matrix generated through the correlation analysis unit 224 and effectively detect lead reversal.

The case classification unit 226 may determine whether the signals included in the input data 210 have lead reversal and classify a lead reversal case based on the correlation matrix generated by the correlation analysis unit 224 or correlation information. For example, the case classification unit 226 may determine whether lead reversal is present by checking the degree of matching between the information contained in the correlation matrix and a predetermined normal case. Furthermore, the case classification unit 226 may classify the lead reversal case in the correlation matrix based on the results of the determination. The case classification unit 226 may classify the lead reversal case present in the input data 210 based on the correlation matrix by using the first neural network model. The first neural network model may include a convolutional neural network configured to receive a visualized matrix and detect a lead reversal case. The first neural network model may be trained based on supervised learning using a normal case matrix, in which no lead reversal is present, as labels. However, the neural network model may be trained based on learning such as unsupervised learning or reinforcement learning other than supervised learning.

Meanwhile, according to an alternative embodiment of the present disclosure, the calculation of the correlation analysis unit 224 and the calculation of the case classification unit 226 may be replaced with a second neural network model. For example, the second neural network model may receive the input data 210 or the output data of the filter unit 222 and classify a lead reversal case. The second neural network model may be trained to extract features for the interpretation of the correlations between signals for the respective leads based on the input data 210 or the output data of the filter unit 22 and classify a lead reversal case based on the features. In this case, the second neural network model may include a neural network configured to perform self-attention-based calculation.

The correction module 230 may correct the electrocardiogram signal, in which an error has been detected by the reversal detection module 220, based on predetermined correction information. The correction module 230 may correct the lead reversal case, detected through the case classification unit 226, according to the correction information. The correction module 230 may use a corresponding signal table (hereinafter referred to as a correction table) for each lead reversal case as the correction information to correct the electrocardiogram signal. The correction table may be information in the form of a table representing the conversion relationship between the electrocardiogram signal of each normal lead and the electrocardiogram signal in which lead reversal has occurred. In this case, a normal state may be understood as a state in which an electrode has been attached to a designated location for normal lead signal measurement. For the correction table, reference may be made to FIG. 4. For example, according to the correction table of FIG. 4, in the case of LA/RA lead reversal in which the left and right arm electrodes are reversed and attached, lead I may show an inverted signal, and lead II and lead III may be changed to each other (Lead I -> -(Lead I), Lead II -> Lead III, and Lead III -> Lead II).

In the table of FIG. 4, "-" may denote a signal with the polarity thereof reversed, and "≒" may denote almost the same signal. CW may denote rotation in the direction RA->LA->LL->RA, and CCW may denote rotation in the direction RA->LL->LA->RA. The correction module 230 may correct an electrocardiogram signal having an error by converting an electrocardiogram signal in which lead reversal has occurred into the electrocardiogram signal of a normal lead based on a correction table including the table of FIG. 4. Meanwhile, although the method of correcting an error in an electrocardiogram signal according to the present disclosure has been described with a focus on the limb leads of 12 leads, the application thereof is not limited to this and may also include 5-, 3- and other-lead electrocardiogram measurement methods.

Referring back to FIG. 2, the output data 240 may be the data obtained by correcting the electrocardiogram signal in which lead reversal has occurred to the electrocardiogram signal of a normal lead. The output data 240 may be transferred to various types of clients and displayed through display devices.

The computing device 200 for correcting an error in an electrocardiogram signal may be used in a form combined with or included in an electrocardiogram signal measurement device, or may be implemented in a separate device.

Furthermore, the correction method performed by the computing device 200 may be performed on a server or computing device located physically away from the electrocardiogram signal measurement device. Furthermore, the correction method performed by the computing device 200 may be implemented in such a manner as to receive measured signals over a network, analyze them, and transmit the results of the analysis back over the network.

FIG. 3 is a diagram illustrating electrocardiogram electrode attachment locations and leads corresponding to these locations according to an embodiment of the present disclosure.

Referring to FIG. 3, standard 12-lead (or 12-lead) electrocardiograms may involve limb leads and precordial leads. The limb leads may include four electrodes (hereinafter referred to as limb electrodes) attached to the limbs. Additionally, the precordial leads may include six electrodes (hereinafter referred to as precordial electrodes) attached to the chest.

The limb electrodes may include a right arm electrode RA, a left arm electrode LA, a right leg electrode RL, and a left leg electrode LL. The right leg electrode RL may be a common electrode or a ground electrode. The limb electrodes may be attached to locations corresponding to the right arm, the left arm, the right leg, and the left leg, respectively.

The precordial electrodes may include a first precordial electrode V1, a second precordial electrode V2, a third precordial electrode V3, a fourth precordial electrode V4, a fifth precordial electrode V5, and a sixth precordial electrode V6.

The limb leads may include standard limb leads I, II, and II and augmented limb leads aVR, aVL, and aVF. Among these limb leads, the standard limb leads are bipolar leads, so that the voltage difference between two electrodes can refer to the trace recorded on an electrocardiogram recorder. For example, a first lead L1 may denote the voltage difference between the right arm electrode RA and the left arm electrode LA, a second lead L2 may denote the voltage difference between the right arm electrode RA and the left leg electrode LL, and a third lead L3 may denote the voltage difference between the left arm electrode LA and the left leg electrode LL. A first electrocardiogram signal or a first channel signal is a signal generated in the first lead L1, a second electrocardiogram signal or a second channel signal is a signal generated in the second lead L2, and a third electrocardiogram signal or a third channel signal is a signal generated in the third lead L3. Among the limb leads, the augmented limb leads are unipolar leads, so that fourth to sixth electrocardiogram signals corresponding to the right arm electrode RA, the left arm electrode LA, and the left leg electrode LL, respectively, can be generated.

The precordial leads are unipolar leads, so that seventh to twelfth electrocardiogram signals corresponding to the precordial electrodes can be generated.

The above description is only one example for standard 12-lead electrocardiogram measurement, so that the present disclosure is not limited to this example.

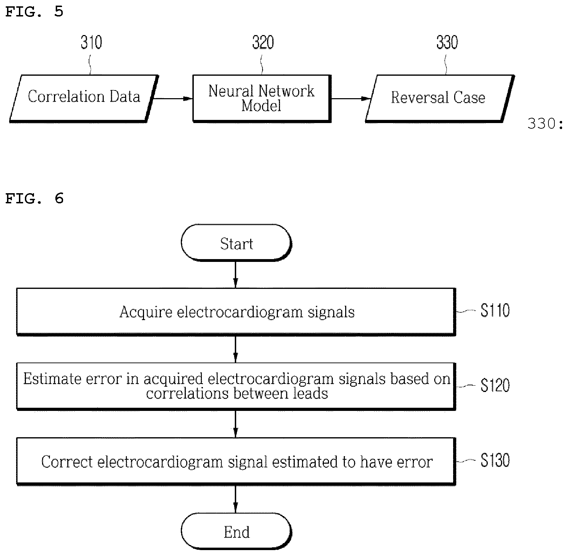

FIG. 5 is a diagram illustrating a neural network model according to an embodiment of the present disclosure.

Referring to FIG. 5, the neural network model 320 for detecting lead reversal may receive correlation data 310 indicating the relationships between all the leads for the measurement of electrocardiogram signals as input and classify a lead reversal case 330 in an electrocardiogram signal. In this case, the correlation data 310 may be a correlation matrix generated in the correlation analysis unit 224 of FIG. 2.

The neural network model 320 may receive a correlation matrix representing the electrocardiogram signal relationships between the leads and interpret the characteristics of the electrocardiogram signal relationships for the individual leads. The neural network model 320 may determine whether lead reversal is present in signals present in the input during a feature analysis process. Additionally, the neural network model 320 may classify lead reversal cases in the input signals based on the results of the determination.

For example, the neural network model 320 may classify lead reversal cases for the limb leads of the 12 leads based on the correlation matrix. For an algorithm for the classification of lead reversal cases for the limb leads of the 12 leads, reference may be made to Equation 2 below:

$$Z = \text{flatten}(A[:3,:]) \in R^{36}$$

$$Y = f(Z, \Theta) \in R^{8} \tag{2}$$

Z may be a correlation matrix for 3 limb leads in electrocardiogram signals for the determination of lead reversal. $\theta$ may be a parameter of a neural network model that classifies lead reversal cases. For example, flatten() may be a function that flattens a multidimensional array space into one dimension.

FIG. 6 is a flowchart showing a method of correcting an error in an electrocardiogram signal according to an embodiment of the present disclosure.

Referring to FIGS. 1 and 6, the computing device 100 according to an embodiment of the present disclosure may acquire electrocardiogram signals in step S110. The computing device 100 may use the electrocardiogram signals measured in the state of being connected to an electrocardiogram measurement device, or may use the electrocardiogram signals transmitted over a network. In this case, the electrocardiogram signals acquired by the computing device 100 may include the electrocardiogram signals measured using a standard 12-lead method, or may include the electrocardiogram signals measured using one of 5-lead, 3-lead, and various-lead methods.

For example, the electrodes attached to the body to acquire electrocardiogram signals may include one or more of a plurality of limb electrodes attached to the distal ends of the limbs and a plurality of precordial electrodes attached to preset locations on the chest according to a standard 12-lead method.

For example, according to the standard 12-lead method, the electrocardiogram signals may include one or more of a first electrocardiogram signal generated based on a first electrode corresponding to the right arm and a second electrode corresponding to the left arm, a second electrocardiogram signal generated based on the first electrode and a third electrode corresponding to the left leg, and a third electrocardiogram signal generated based on the second electrode and the third electrode.

The computing device 100 may estimate an error in the electrocardiogram signals based on the correlations between leads in step S120. The computing device 100 may generate a correlation matrix representing the electrocardiogram signal relationships between the leads based on the acquired electrocardiogram signals. Additionally, the computing device 100 may detect the electrocardiogram signal in which lead reversal has occurred among the acquired electrocardiogram signals based on the correlation matrix. In the process of calculating the correlations between electrocardiogram signals for the individual leads, the computing device 100 may preprocess the input signals or generate a correlation matrix by considering weights or the like.

In the process of detecting the electrocardiogram signal in which lead reversal has occurred among the acquired electrocardiogram signals based on the correlation matrix, the computing device 100 may classify the electrocardiogram signal in which lead reversal has occurred among the acquired electrocardiogram signals by inputting the correlation matrix to a pre-trained first neural network model. The computing device 100 may classify the electrocardiogram signal in which lead reversal has occurred among the electrocardiogram signals by inputting the electrocardiogram signal to a pre-trained self-attention-based second neural network model.

The computing device 100 may extract target signals for the analysis of the correlations from the acquired electrocardiogram signals by using a band-pass filter to remove noise from the electrocardiogram signals.

The computing device 100 may correct the electrocardiogram signal estimated to have an error and provide it to a user in step S130. The computing device 100 may correct the electrocardiogram signal estimated to be related to lead reversal by using a correction table representing the conversion relationships between the electrocardiogram signals of respective normal leads and the electrocardiogram signals in each of which lead reversal has occurred. For example, the correction table may include the table illustrated in FIG. 4.

Through the method of correcting an error in an electrocardiogram signal according to an embodiment of the present disclosure, even when lead reversal has occurred due to the misplacement of an electrocardiogram electrode, information about the misplaced electrode and a lead reversal case may be accurately determined. Additionally, the method of correcting an error in an electrocardiogram signal according to an embodiment of the present disclosure, electrocardiogram signals may be rapidly provided without the re-attachment of an electrode or the re-measurement of signals.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of correcting an error in an electrocardiogram signal, the method being performed by a computing device including at least one processor, the method comprising:

   acquiring electrocardiogram signals;
   estimating an error in the acquired electrocardiogram signals based on correlations between leads for measurement of the acquired electrocardiogram signals; and
   correcting an electrocardiogram signal, estimated to have the error, based on correction information for correction of an error in an electrocardiogram signal.

2. The method of claim 1, wherein estimating the error in the acquired electrocardiogram signals comprises:

   generating a correlation matrix representing electrocardiogram signal relationships between the leads based on the acquired electrocardiogram signals; and
   detecting an electrocardiogram signal, in which lead reversal has occurred, among the acquired electrocardiogram signals based on the correlation matrix.

3. The method of claim 2, wherein detecting the electrocardiogram signal in which lead reversal has occurred comprises classifying the electrocardiogram signal, in which the lead reversal has occurred, among the acquired electrocardiogram signals by inputting the correlation matrix to a pre-trained first neural network model.

4. The method of claim 1, wherein estimating the error in the acquired electrocardiogram signals comprises classifying the electrocardiogram signal, in which the lead reversal has occurred, among the electrocardiogram signals by inputting the electrocardiogram signals to a pre-trained second neural network model.

5. The method of claim 1, wherein estimating the error in the acquired electrocardiogram signals comprises extracting target signals for analysis of the correlations from the acquired electrocardiogram signals by using a band-pass filter.

6. The method of claim 1, wherein the correction information comprises a correction table representing conversion relationships between electrocardiogram signals of normal leads and electrocardiogram signals in which lead reversal has occurred.

7. The method of claim 1, wherein electrodes attached to a human body to acquire the electrocardiogram signals comprise one or more of a plurality of electrodes attached to distal ends of limbs and a plurality of electrodes attached to preset locations on a chest.

8. The method of claim 1, wherein the acquired electrocardiogram signals comprise one or more of a first electrocardiogram signal generated based on a first electrode corresponding to a right arm and a second electrode corresponding to a left arm, a second electrocardiogram signal generated based on the first electrode and a third electrode corresponding to a left leg, a third electrocardiogram signal generated based on the second electrode and the third electrode, and precordial electrocardiogram signals corresponding to a plurality of precordial electrodes, respectively.

9. A computer program stored in a computer-readable storage medium, the computer program performing operations of correcting an error in an electrocardiogram signal when executed on one or more processors, wherein the operations comprise operations of:

   acquiring electrocardiogram signals;
   estimating an error in the acquired electrocardiogram signals based on correlations between leads for measurement of the acquired electrocardiogram signals; and
   correcting an electrocardiogram signal, estimated to have the error, based on correction information for correction of an error in an electrocardiogram signal.

10. A computing device for correcting an error in an electrocardiogram signal, the computing device comprising:

    a processor including at least one core;
    memory including program codes that are executable on the processor; and
    a network unit configured to acquire electrocardiogram signals;
    wherein the processor acquires electrocardiogram signals, estimates an error in the acquired electrocardiogram signals based on correlations between leads for measurement of the acquired electrocardiogram signals, and corrects an electrocardiogram signal, estimated to have the error, based on correction information for correction of an error in an electrocardiogram signal.

FIG. 1

<u>100</u>

Processor ~ 110

Memory ~ 120

Network Unit ~ 130

FIG. 2

200

Input Data — 210

220

Reversal Detection Module

Filter Unit — 222

Correlation Analysis Unit — 224

Case Classification Unit — 226

230

Collection Module

Output Data — 240

FIG. 3

FIG. 4

| Reversal Case | Lead Signal | | | | | |
|---|---|---|---|---|---|---|
| | I | II | III | AVR | AVL | AVF |
| LA/RA | − I | III | II | AVR | AVL | AVF |
| LA/LL | II | I | − III | AVR | AVF | AVL |
| RA/LL | − III | − II | − I | AVF | AVL | AVR |
| CW | III | − I | − II | AVL | AVF | AVR |
| CCW | − II | − III | I | AVF | AVR | AVL |
| RA/RL | ≒ − III | ≒ 0 | III | ≒ AVF | ≒ − III | ≒ AVR |
| LA/RL | ≒ II | II | ≒ 0 | ≒ − II | ≒ AVF | ≒ AVL |
| LL/RL | I | ≒ II | ≒ III | ≒ AVR | ≒ AVL | ≒ AVF |
| RA/RL | ≒ 0 | − II | − I | ≒ AVL | ≒ AVR | ≒ − III |
| LA/LL | ≒ 0 | − II | − I | ≒ AVL | ≒ AVR | ≒ − III |

FIG. 5

310                       320                       330

| Correlation Data | → | Neural Network Model | → | Reversal Case |

330:

FIG. 6

```
        ┌──────────┐
        │   Start   │
        └──────────┘
              │
              ▼
  ┌────────────────────────────────────────┐
  │   Acquire electrocardiogram signals     │──── S110
  └────────────────────────────────────────┘
              │
              ▼
  ┌────────────────────────────────────────┐
  │ Estimate error in acquired electrocardiogram signals based on │──── S120
  │         correlations between leads       │
  └────────────────────────────────────────┘
              │
              ▼
  ┌────────────────────────────────────────┐
  │ Correct electrocardiogram signal estimated to have error │──── S130
  └────────────────────────────────────────┘
              │
              ▼
        ┌──────────┐
        │    End    │
        └──────────┘
```

## INTERNATIONAL SEARCH REPORT

| International application No. |
| --- |
| **PCT/KR2022/014204** |

**A.    CLASSIFICATION OF SUBJECT MATTER**

**A61B 5/276**(2021.01)i; **A61B 5/327**(2021.01)i; **A61B 5/319**(2021.01)i; **A61B 5/346**(2021.01)i; **A61B 5/28**(2021.01)i; **A61B 5/00**(2006.01)i

According to International Patent Classification (IPC) or to both national classification and IPC

**B.    FIELDS SEARCHED**

Minimum documentation searched (classification system followed by classification symbols)

A61B 5/276(2021.01); A61B 5/00(2006.01); A61B 5/04(2006.01); A61B 5/0402(2006.01); A61B 5/0408(2006.01); A61B 5/0452(2006.01); A61B 5/046(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심전도(ECG, ElectroCardioGram), 리드 역전(lead reversal), 신호복원(signal restoring), 필터(filter), 오류(error)

**C.    DOCUMENTS CONSIDERED TO BE RELEVANT**

| Category* | Citation of document, with indication, where appropriate, of the relevant passages | Relevant to claim No. |
| --- | --- | --- |
| X | KR 10-2010-0004239 A (SONG, Meong Gun et al.) 13 January 2010 (2010-01-13)<br>See paragraphs [0006]-[0034]; and figures 1-3. | 1,9-10 |
| Y | | 2-8 |
| Y | US 2020-0138316 A1 (ALIVECOR, INC.) 07 May 2020 (2020-05-07)<br>See paragraphs [0019]-[0038]. | 2-3,5 |
| Y | CAO, Zheng et al. REVERSAL NO LONGER MATTERS: ATTENTION-BASED ARRHYTHMIA DETECTION WITH LEAD-REVERSAL ECG DATA. ICASSP 2020 - 2020 IEEE International Conference on Acoustics, Speech and Signal Processing (ICASSP). pp. 951-955, 04 May 2020. [Retrieved on 20 December 2022]. Retrieved from <https://doi.org/10.1109/ICASSP40776.2020.9053307>.<br>See page 951, left column, line 2 – page 952, left column, line 29; and table 1. | 3-4,6-8 |
| A | KR 10-2005-0119890 A (SAMSUNG ELECTRONICS CO., LTD.) 22 December 2005 (2005-12-22)<br>See paragraphs [0028]-[0085]; and figures 4-18. | 1-10 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 January 2023** | **11 January 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

<table>
<tr><td colspan="2"></td><td>International application No.<br>**PCT/KR2022/014204**</td></tr>
</table>

**C. DOCUMENTS CONSIDERED TO BE RELEVANT**

| Category* | Citation of document, with indication, where appropriate, of the relevant passages | Relevant to claim No. |
|---|---|---|
| A | KR 10-2015-0020955 A (KWANGWOON UNIVERSITY INDUSTRY-ACADEMIC COLLABORATION FOUNDATION) 27 February 2015 (2015-02-27)<br>See paragraphs [0001]-[0031]; and figures 1-6. | 1-10 |

Form PCT/ISA/210 (second sheet) (July 2022)

**INTERNATIONAL SEARCH REPORT**
Information on patent family members

International application No.

**PCT/KR2022/014204**

| Patent document cited in search report | | | Publication date (day/month/year) | Patent family member(s) | | | Publication date (day/month/year) |
|---|---|---|---|---|---|---|---|
| KR | 10-2010-0004239 | A | 13 January 2010 | CN | 102083363 | A | 01 June 2011 |
| | | | | KR | 10-0951448 | B1 | 07 April 2010 |
| | | | | WO | 2010-002171 | A2 | 07 January 2010 |
| US | 2020-0138316 | A1 | 07 May 2020 | US | 09254095 | B2 | 09 February 2016 |
| | | | | US | 10478084 | B2 | 19 November 2019 |
| | | | | US | 11103176 | B2 | 31 August 2021 |
| | | | | US | 2014-128758 | A1 | 08 May 2014 |
| | | | | US | 2016-249823 | A1 | 01 September 2016 |
| | | | | US | 2021-290141 | A1 | 23 September 2021 |
| | | | | WO | 2014-074913 | A1 | 15 May 2014 |
| KR | 10-2005-0119890 | A | 22 December 2005 | CN | 1736326 | A | 22 February 2006 |
| | | | | CN | 1736326 | C | 22 February 2006 |
| | | | | JP | 2006-000658 | A | 05 January 2006 |
| | | | | JP | 4208862 | B2 | 14 January 2009 |
| | | | | KR | 10-0624425 | B1 | 19 September 2006 |
| | | | | US | 08606353 | B2 | 10 December 2013 |
| | | | | US | 2006-0009691 | A1 | 12 January 2006 |
| KR | 10-2015-0020955 | A | 27 February 2015 | KR | 10-1524226 | B1 | 29 May 2015 |

Form PCT/ISA/210 (patent family annex) (July 2022)